# FASCICULE DE BREVET EUROPEEN

(11) **EP 0 576 379 B1**
(45) Date de publication et mention de la délivrance du brevet: **11.09.1996**
(21) Numéro de dépôt: 93420274.8
(22) Date de dépôt: 24.06.1993
(51) Int. Cl.: A61B 17/60, F16F 1/36

(54) **Utilisation d'amortisseurs perfectionnés à limite de déplacement dans un dispositif pour la stabilisation intervertébrale**
Verwendung von Dämpfern mit verbesserter Endlagendämpfung in einer Vorrichtung zur Stabilisierung der Zwischenwirbelräume
Use of dampers with improved damping at the end of stroke in a device for intervertebral stabilisation

(30) Priorité: 25.06.1992 FR 9208068
(43) Date de publication de la demande: 29.12.1993
(73) Titulaire: PSI, 69002 Lyon (FR)
(72) Inventeur: Navas, Fernand, F-69260 Charbonnieres-les-Bains (FR)
(74) Mandataire: Karmin, Roger

(56) Documents cités:
- EP-A- 0 478 470
- EP-A- 0 516 567
- BE-A- 629 690
- DE-U- 1 827 221
- FR-A- 2 139 332
- GB-A- 1 227 359
- NL-A- 279 186
- US-A- 3 901 495
- US-A- 3 904 226

## Description

La présente invention concerne l'utilisation de certains amortisseurs comprenant des moyens de résister progressivement de façon exponentielle à l'avance d'un piston sous l'effet d'une force de compression axiale, pour la réalisation d'un dispositif de stabilisation intervertébral.

De tels amortisseurs sont bien connus dans le domaine de l'automobile. Par exemple les brevets US-3 904 226 et NL-279 186 décrivent chacun un amortisseur à simple ou à double effet qui soit susceptible de résister progressivement de façon exponentielle à l'avance du piston, de telle manière qu'après une certaine course du piston, l'amortisseur s'oppose à tout déplacement du piston au-delà d'une valeur déterminée de ce déplacement.

A cet effet, l'amortisseur renferme entre le fond du cylindre et le piston un corps élastique dont le volume est inférieur à celui de la chambre déterminée par la position à l'état libre du piston par rapport au fond du cylindre.

Un tel amortisseur est dit à simple effet, c'est-à-dire qu'il ne fonctionne que dans un seul sens. Si l'on désire un amortisseur à double effet, c'est-à-dire agissant dans deux sens opposés, il suffit de placer un second corps élastique dans le compartiment situé entre le piston et le couvercle fermant ledit cylindre à l'opposé de son fond, ce second corps élastique comportant un volume inférieur à celui du compartiment précité.

La présente invention concerne donc l'utilisation d'un amortisseur du genre comprenant des moyens de résister progressivement de façon exponentielle à l'avance d'un piston sous l'effet d'une force de compression axiale, lesdits moyens constituant une butée s'opposant à tout déplacement du piston au-delà d'une valeur déterminée de ce déplacement, pour la réalisation d'un dispositif de stabilisation intervertébral.

L'amortisseur comprend d'autres moyens pour résister progressivement de façon exponentielle au recul du piston sous l'effet d'une force d'extension de direction opposée à celle de compression.

Les moyens pour résister à tout déplacement progressif du piston consistent en au moins un corps élastique dont le volume est inférieur à celui de la chambre du cylindre dans lequel il est enfermé.

Ainsi, la variation du volume de la chambre ou du compartiment entraîne une déformation du corps élastique correspondant à laquelle s'oppose l'indéformabilité de leur paroi, de telle sorte qu'une force antagoniste s'oppose progressivement au déplacement du piston jusqu'à l'arrêter lorsque cette force devient exponentielle.

Le dessin annexé, donné à titre d'exemple, permettra de mieux comprendre l'invention, les caractéristiques qu'elle présente et les avantages qu'elle est susceptible de procurer :
Fig. 1 est une coupe longitudinale d'un amortisseur suivant l'invention prévu à simple effet.
Fig. 2 est une vue semblable à celle de fig. 1, mais illustrant un amortisseur à double effet.
Fig. 3 est une courbe illustrant les variations de la force antagoniste développée par l'amortisseur de fig. 1 et 2.
Fig. 4 est une variante de réalisation du dispositif de fig. 2. On a fait figurer en pointillés les éléments permettant de l'associer à deux vertèbres.

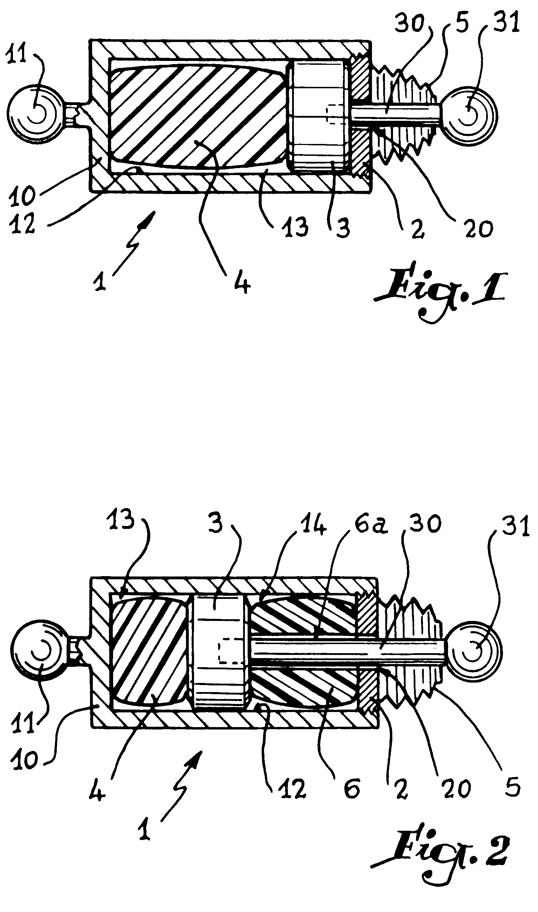

On a représenté en fig. 1 un amortisseur suivant l'invention comprenant essentiellement un cylindre 1 dont la face extérieure du fond 10 est solidaire d'une rotule 11, ce cylindre étant fermé au niveau de son extrémité opposée au fond 10 par un couvercle 2. Avant le montage de ce couvercle, on engage à l'intérieur du cylindre 1 un piston 3 solidaire par une tige 30 d'une rotule 31, la tige 30 traversant un trou central 20 du couvercle 2. On observe que la hauteur du piston 3 est importante par rapport à la longueur du cylindre 1, de manière à assurer un bon guidage du piston dans l'alésage 12 dudit cylindre lorsque ce piston se déplace dans cet alésage.

Préalablement à l'introduction du piston 3 dans l'alésage 12 du cylindre 1, on place contre la face intérieure du fond 10 un corps élastique 4 tel qu'un bloc de caoutchouc naturel ou synthétique dont le volume à l'état libre est légèrement inférieur à celui de la chambre 13, déterminée par le volume intérieur du cylindre 1 défini entre son fond 10 et le piston 3 lorsque celui-ci repose contre le couvercle 2. Un soufflet 5 solidaire du couvercle 2 et de la tige 30 assure l'étanchéité au niveau de la traversée de cette tige dans le trou 20 de ce couvercle.

Lorsqu'une force agit sur les rotules 11 et 31 en direction l'une de l'autre (compression), elle provoque une translation du piston 3 à partir de sa position de repos dans laquelle il est en appui contre la face interne du couvercle 2 en direction du fond 10 du cylindre 1. Ce déplacement entraîne une déformation du corps élastique 4, laquelle est contrecarrée par la rigidité des parois du cylindre 1, de telle sorte que ce corps 4 s'oppose au déplacement du piston 3 avec une réaction croissante. Lorsque le volume de la chambre 12 est égal à celui du corps élastique 4 déjà comprimé, la réaction développée par ce corps à l'encontre du déplacement du piston 3 devient exponentielle et à la limite forme une butée interdisant tout déplacement subséquent du piston 3, si bien qu'en fait le déplacement de celui-ci est alors stoppé. Bien entendu, l'amortisseur représenté en fig. 1 ne fonctionne que dans le sens de la compression, c'est-à-dire lorsqu'une force est appliquée pour rapprocher les rotules 11 et 31.

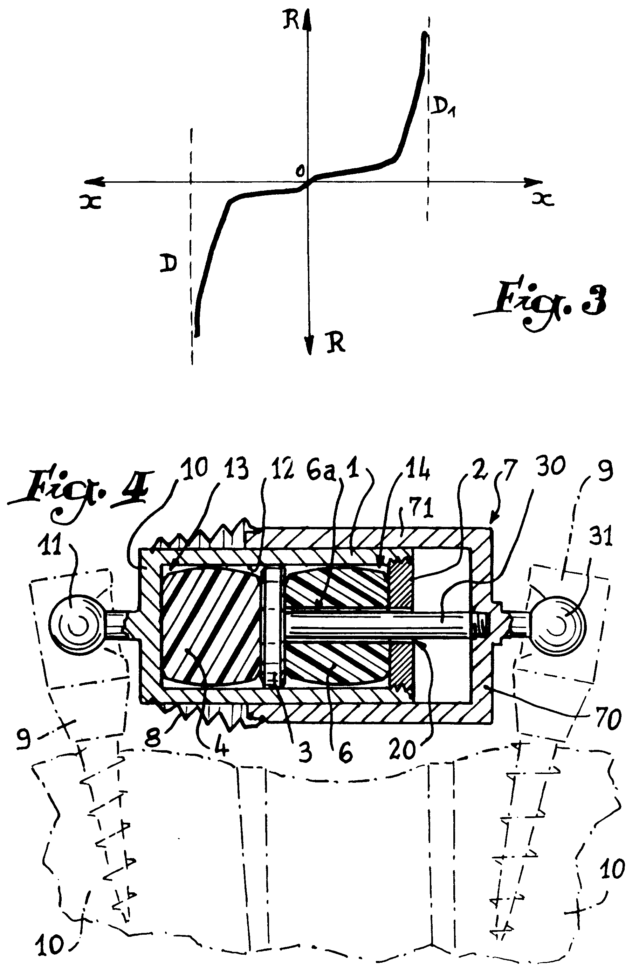

Dans le mode d'exécution de fig. 2, à l'état libre, le piston 3 n'est pas en appui contre le couvercle 2, mais il forme avec ce dernier un compartiment 14 dans lequel on place un bloc élastique 6 comportant un trou central 6a qui est traversé par la tige 30 du piston 3. Là encore, le volume du second corps élastique 6 est légèrement inférieur, à l'état libre, à celui du compartiment 14.

Le fonctionnement est rigoureusement le même que celui de l'amortisseur illustré en fig. 1 lorsqu'il résiste à une force de compression tendant à rapprocher les rotules 11 et 31. De manière identique, lorsqu'une force d'extension est appliquée à l'amortisseur de fig. 2, c'est-à-dire lorsque cet effort tend à éloigner les rotules 11 et 31, le piston comprime le corps élastique 6 qui résiste à son déplacement de manière de plus en plus importante au fur et à mesure que le volume du corps élastique 6 s'approche de celui du compartiment 14. Puis la réaction devient exponentielle. Elle forme, lorsque le second corps élastique 6 devient incompressible, une butée d'arrêt qui limite tout déplacement ultérieur du piston 3.

On a représenté en fig. 3 la courbe qui illustre la variation de la force résistante de l'amortisseur de fig. 2. La partie gauche de la courbe correspond à un effort de compression appliqué sur l'amortisseur et qui représente en fait un déplacement négatif illustré à partir de l'origine. La réaction R croît pour la majorité du déplacement de manière relativement faible par rapport à ce déplacement, puis devient exponentielle pour finir asymptotique à une droite D parallèle à l'axe R des ordonnées.

Si, au contraire, le déplacement de l'amortisseur est positif (cas d'une traction), la courbe illustrant la résistance antagoniste de l'amortisseur est pratiquement symétrique par rapport à celle correspondant à une compression, cette partie de la courbe devenant asymptotique à une droite D1 également parallèle à l'axe R des ordonnées.

Bien entendu, la courbe est symétrique si les deux corps élastiques 4 et 6 présentent les mêmes caractéristiques de volume et de flexibilité et si la chambre 13 et le compartiment 14 sont de même volume. Par contre, on peut faire varier et les caractéristiques des corps élastiques 4 et 6 et les volumes de la chambre 13 et du compartiment 14 de manière à obtenir des efforts résistants différents à la compression ou à la traction.

On a enfin présenté en fig. 4 un autre mode d'exécution de l'amortisseur de fig. 2. On observe sur cette figure ainsi que sur la fig. 1 que le piston 3 présente une largeur importante afin d'assurer son guidage dans l'alésage du cylindre 1. Fig. 4 montre une autre façon d'assurer le guidage en question. Dans ce mode d'exécution, le piston 3 est de faible épaisseur, tandis que sa tige 30 est solidaire d'une cage tubulaire 7 par l'intermédiaire de son fond 70, la jupe 71 de cette cage coulissant juste par rapport à l'extérieur du cylindre 1. Un soufflet 8 semblable à celui 5 relie le cylindre 1 et le bas de la jupe 71 de la cage 7 pour assurer l'étanchéité.

On a illustré en fig. 4 deux implants 9 dont la tête est montée articulée par rapport aux rotules 11 et 31, tandis que leur corps est vissé dans deux vertèbres 10 de manière à constituer un dispositif de stabilisation intervertébral fonctionnant en parallèle et de la même manière que le disque séparant les deux vertèbres.

En effet le dispositif suivant l'invention n'est pas destiné à remplacer le disque intervertébral. Le dispositif est prévu pour soulager le disque lorsque celui-ci supporte des efforts de compression et de traction.

Bien entendu, on peut prévoir que le piston 3 ne soit jamais totalement libre et toujours maintenu stable par l'existence de forces spontanées et opposées développées par les deux corps élastiques 4 et 6. Ces forces n'étant pas nulles au point d'équilibre, on obtient ainsi une stabilisation élastique du piston 3 à un point d'équilibre, de telle sorte que tout déplacement en traction ou en compression excercé sur l'amortisseur entraîne immédiatement une réaction antagoniste. Cette pré-contrainte initiale peut bien entendu être fixée en faisant varier le volume et les caractéristiques des corps élastiques dans un amortisseur dont la chambre et le compartiment restent de volume constant.

Comme indiqué plus haut une utilisation particulièrement avantageuse de l'amortisseur suivant l'invention consiste à la création d'un dispositif de stabilisation intervertébral, toutefois différentes autres utilisations médicales peuvent être envisagées par exemple en combinaison avec un système prothétique quel qu'il soit, l'amortisseur pouvant être utilisé sous les formes d'exécution de fig. 1 (simple effet) ou fig. 2 et 4 (double effet).

Il doit d'ailleurs être entendu que la description qui précède n'a été donnée qu'à titre d'exemple et qu'elle ne limite nullement le domaine de l'invention dont on ne sortirait pas en remplaçant les détails d'exécution décrits par tous autres équivalents. En particulier, on pourrait prévoir de placer une coupelle contre chaque corps élastique 4, 6 et placer entre chaque coupelle et le fond du cylindre, respectivement le couvercle, un ressort non représenté augmentant la réaction développée par l'amortisseur à la compression ou à l'extension ou sous les deux contraintes.

## Revendications

1. Utilisation d'un amortisseur perfectionné du genre comprenant des moyens de résister progressivement de façon exponentielle à l'avance d'un piston sous l'effet d'une force de compression axiale, lesdits moyens constituant une butée s'opposant à tout déplacement du piston au-delà d'une valeur déterminée de ce déplacement, à un dispositif de stabilisation intervertébral.

2. Utilisation suivant la revendication 1, caractérisée en ce que l'amortisseur comprend d'autres moyens (6) de résister progressivement de façon exponentielle au recul du piston (3) sous l'effet d'une force d'extension de direction opposée à celle de compression.

3. Utilisation suivant la revendication 1, caractérisée en ce que la chambre (13) limitée par le fond (10) du cylindre (1) et le piston (3) en position libre contient un corps élastique (4) dont le volume est inférieur à celui de ladite chambre, la compression totale du corps élastique (4) déterminant la course du piston (3) sous l'effet d'une force de compression.

4. Utilisation suivant la revendication 2, caractérisée en ce qu'un second corps élastique (6) est placé dans le compartiment (14) du cylindre engendré entre le piston (3) et le couvercle (2) fermant ledit cylindre (1) à l'opposé de son fond (10), le volume du second corps élastique (6) étant inférieur à celui dudit compartiment (14) afin que la course du piston (3) sous l'effet d'une force opposée à celle de compression soit déterminée par la compression totale du corps 6.

5. Utilisation suivant la revendication 4, caractérisée en ce que le cylindre (1) coulisse dans une cuve (7) dont le fond (70) est assemblé au piston (3).

6. Utilisation suivant la revendication 1, caractérisée en ce que les corps élastiques (4, 6) développent au repos une force antagoniste initiale par rapport au piston (3).

## Claims

1. The use of an improved shock-absorber of the type comprising means for progressively resisting exponentially the advance of a piston under the action of an axial compressive load, said means forming a stop resisting any displacement of the piston beyond a given value of this displacement, in an intervertebral stabilisation device.

2. The use according to Claim 1, characterised in that the shock-absorber comprises other means (6) for progressively resisting exponentially the recoil of the piston (3) under the action of an extension force in a direction opposite to that of the compressive load.

3. The use according to Claim 1, characterised in that the chamber (13) defined by the base (10) of the cylinder (1) and the piston (3) in the free position contains an elastic body (4), the volume of which is less than that of said chamber, the total compression of the elastic body (4) determining the stroke of the piston (3) under the action of a compressive load.

4. The use according to Claim 2, characterised in that a second elastic body (6) is placed in the compartment (14) of the cylinder formed between the piston (3) and the cover (2), closing said cylinder (1) opposite the base (10) thereof, the volume of the second elastic body (6) being less than that of said compartment (14) so that the stroke of the piston (3) under the action of a force opposed to that of the compressive load is determined by the total compression of the body (6).

5. The use according to Claim 4, characterised in that the cylinder (1) slides in a cell (7), the base (70) of which is joined to the piston (3).

6. The use according to Claim 1, characterised in that the elastic bodies (4, 6), at rest, develop an initial opposed force relative to the piston (3).

## Patentansprüche

1. Verwendung eines verbesserten Dämpfers des Typs, der Mittel zum zunehmenden Widerstehen in exponentieller Weise gegen das Vorrücken eines Kolbens unter Wirkung einer axialen Druckkraft umfaßt, wobei die Mittel einen Anschlag bilden, der sich jeglicher Verschiebung des Kolbens über einen bestimmten Wert dieser Verschiebung hinaus entgegenstellt, für eine Vorrichtung zur Zwischenwirbelstabilisierung.

2. Verwendung nach Anspruch 1, dadurch gekennzeichnet, daß der Dämpfer andere Mittel (6) zum zunehmenden Widerstehen in exponentieller Weise gegen den Rückschub des Kolbens (3) unter der Wirkung einer Dehnungskraft in den der Druckkraft entgegengesetzten Richtung umfaßt.

3. Verwendung nach Anspruch 1, dadurch gekennzeichnet, daß die von dem Boden (10) des Zylinders (1) und dem Kolben (3) in freier Position begrenzte Kammer (13) einen elastischen Körper (4) enthält, dessen Volumen kleiner als das der Kammer ist, wobei die Gesamtkompression des elastischen Körpers (4) den Weg des Kolbens (3) unter der Wirkung einer Druckkraft bestimmt.

4. Verwendung nach Anspruch 2, dadurch gekennzeichnet, daß ein zweiter elastischer Körper (6) in den Raum (14) des Zylinders zwischen dem Kolben (3) und dem den Zylinder (1) entgegengesetzt zu dem Boden (10) abschließenden Deckel (2) angeordnet ist, wobei das Volumen des zweiten elastischen Körpers (6) kleiner als das des Raums (14) ist, damit der Weg des Kolbens unter der Wirkung einer der Kompression entgegengesetzten Kraft durch die Gesamtkompression des Körpers (6) bestimmt wird.

5. Verwendung nach Anspruch 4, dadurch gekennzeichnet, daß der Zylinder (1) in einer Küvette (7) gleitet, deren Boden (70) mit dem Kolben (3) zusammengefügt ist.

6. Verwendung nach Anspruch 1, dadurch gekennzeichnet, daß die elastischen Körper (4,6) in Ruhe eine Ausgangsgegenkraft in bezug auf den Kolben (3) entwickeln.
